(19) Europäisches Patentamt
European Patent Office
Office européen des brevets

(11) Numéro de publication : **0 526 314 A1**

(12) **DEMANDE DE BREVET EUROPEEN**

(21) Numéro de dépôt : **92402134.8**

(22) Date de dépôt : **23.07.92**

(51) Int. Cl.$^5$ : **C07D 207/40,** C08G 73/02

(30) Priorité : **31.07.91 FR 9109847**

(43) Date de publication de la demande :
**03.02.93 Bulletin 93/05**

(84) Etats contractants désignés :
**AT BE CH DE DK ES GB GR IT LI LU NL PT SE**

(71) Demandeur : **ELF ANTAR FRANCE**
**Tour Elf, Place de la Coupole**
**F-92078 Paris La Defense (FR)**

(72) Inventeur : **Garapon, Jacques**
**120, Rue Mazenod**
**F-69005 Lyon (FR)**
Inventeur : **Bregent, Roger,**
**3, Sente de l'Aunay**
**F-78250 Oinville Sur Montcient (FR)**
Inventeur : **Touet, Rémi**
**Residence Mutualiste, Le Fontanil**
**F-38120 Saint Egreve (FR)**
Inventeur : **Mulard, Philippe**
**36, avenue de Mozart**
**F-69780 St Pierre de Chandieu (FR)**
Inventeur : **Schmelzle, Pierre**
**8, avenue de Colombier**
**F-42200 St Julien Molin Molette (FR)**

(74) Mandataire : **Andreeff, François**
**INSTITUT FRANCAIS DU PETROLE 4, avenue de Bois-Préau**
**F-92502 Rueil-Malmaison (FR)**

(54) **Composés polyazotés comportant deux cycles terminaux de type imide, leurs préparations et leurs utilisations.**

(57) Composé polyazoté, comportant deux cycles terminaux de type imide, sa préparation par des méthodes classiques de la chimie organique et son utilisation comme additif pour carburant moteur. Ce composé répond à la formule générale (I) :

(I)

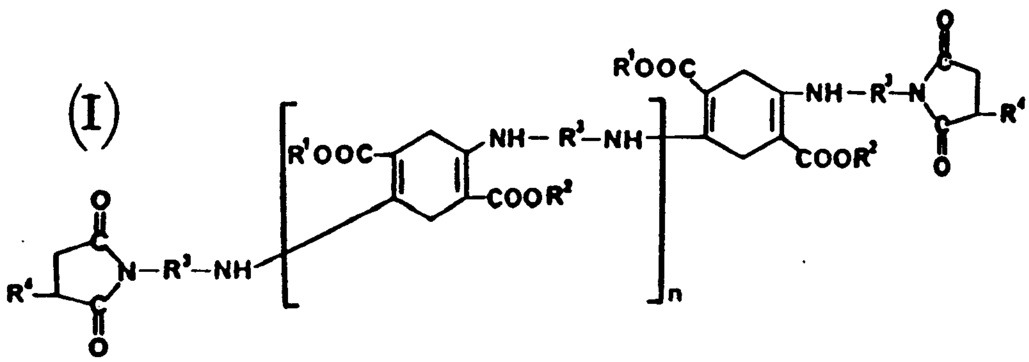

dans laquelle $R^1$ et $R^2$, identiques ou différents, représentent chacun un groupe hydrocarboné ayant de 1 à 120 atomes de carbone ou un groupe de formule $R^5$-(-O-$R^6$-)$_a$-(-O$R^7$-)$_b$- dans laquelle $R^6$ et $R^7$, identiques ou différents, représentent chacun un groupe hydrocarboné divalent ayant de 2 à 6 atomes de carbone, $R^5$ représente un groupe hydrocarboné monovalent ayant de 1 à 60 atomes de carbone ; a est zéro ou un nombre entier de 1 à 100 et b est un nombre entier de 1 à 100 ; $R^3$ est un groupe hydrocarboné divalent ayant de 2 à 60 atomes de carbone ou un groupe divalent de formule

$$-R^8-(-X-R^9-)_c-(X-R^{10}-)_d-(-X-R^{11}-)_e-$$

dans laquelle X est choisi parmi les groupes -O- et -$NR^{12}$-, $R^{12}$ représentant un atome d'hydrogène ou un groupe hydrocarboné ayant de 1 à 6 atomes de carbone, $R^8$, $R^9$, $R^{10}$ et $R^{11}$, identiques ou différents, représentent chacun un groupe hydrocarboné divalent ayant de 2 à 6 atomes de carbone, c est un nombre entier de 1 à 120, d et e, identique ou différents, sont chacun zéro ou un nombre entier de 1 à 120 et la somme c + d + e est un nombre entier de 1 à 120 ; $R^4$ est un atome d'hydrogène ou un groupe hydrocarboné ayant de 1 à 200 atomes de carbone et n est un nombre de 0 à 20.

EP 0 526 314 A1

La présente invention concerne des composés polyazotés, comportant deux cycles terminaux de type imide, leurs procédés de préparation et leurs utilisations. Ces composés comportent habituellement dans leurs formules au moins deux atomes d'azotes en plus des atomes d'azote des cycles imides et répondent à la formule générale (I) suivante :

(I)

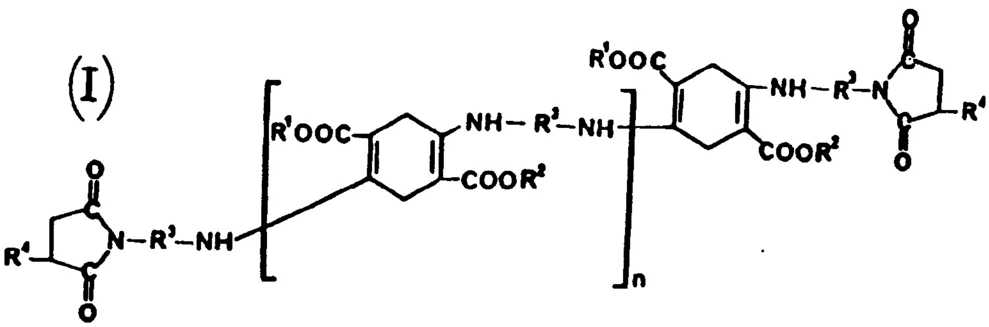

dans laquelle $R_1$ et $R_2$, identiques ou différents, représentent chacun un groupe hydrocarboné monovalent ayant de 1 à 120 atomes de carbone, ou un groupe de formule $R^5\text{-}(\text{-O-}R^6\text{-})_a\text{-}(\text{-O}R^7\text{-})_b\text{-}$ dans laquelle $R^6$ et $R^7$, identiques ou différents, représentent chacun un groupe hydrocarboné divalent ayant de 2 à 6 atomes de carbone, $R^5$ est un groupe hydrocarboné monovalent ayant de 1 à 60 atomes de carbone; a est zéro ou un nombre entier de 1 à 100 et b est un nombre entier de 1 à 100 ; $R^3$ est un groupe hydrocarboné divalent ayant de 2 à 60 atomes de carbone ou un groupe divalent de formule $\text{-}R^8\text{-}(\text{-X-}R^9\text{-})_c\text{-}(\text{-X-}R^{10}\text{-})_d\text{-}(\text{-X-}R^{11}\text{-})_e\text{-}$ dans laquelle X est choisi parmi les groupes -O- et $\text{-N}R^{12}\text{-}$, $R^{12}$ représentant un atome d'hydrogène ou un groupe hydrocarboné ayant de 1 à 6 atomes de carbone, $R^8$,$R^9$,$R^{10}$ et $R^{11}$, identiques ou différents, représentent chacun un groupe hydrocarboné divalent ayant de 2 à 6 atomes de carbone, c est un nombre entier de 1 à 120, d et e, identiques ou différents, sont chacun zéro ou un nombre entier de 1 à 120 et la somme c + d + e est un nombre entier de 1 à 120, $R^4$ est un atome d'hydrogène ou un groupe hydrocarboné monovalent ayant de 1 à 200 atomes de carbone et n est un nombre de 0 à 20.

Dans la formule générale (I) ci-avant, $R^1$ et $R^2$, identiques ou différents, représentent chacun le plus souvent un groupe aliphatique, saturé ou insaturé, linéaire ou ramifié ayant de 1 à 60 atomes de carbone et par exemple un groupe alkyle, linéaire ou ramifié ayant de 1 à 30 atomes de carbone ou un groupe de formule $R^5\text{-}(\text{-O-}R^6\text{-})_a\text{-}(\text{-O}R^7\text{-})_b\text{-}$ dans laquelle $R^6$ et $R^7$, identiques ou différents, représentent chacun le plus souvent un groupe aliphatique divalent, saturé ou insaturé, linéaire ou ramifié ayant de 2 à 4 atomes de carbone et par exemple un groupe alkylène, linéaire ou ramifié ayant de 2 à 4 atomes de carbone tel que par exemple un groupe éthylène, triméthylène, propylène, tétraméthylène et isobutylène, $R^5$ représente le plus souvent un groupe aliphatique monovalent, saturé ou insaturé, linéaire ou ramifié ayant de 1 à 20 atomes de carbone et par exemple un groupe alkyle, linéaire ou ramifié ayant de 1 à 20 atomes de carbone ; a est le plus souvent zéro ou un nombre entier de 1 à 50 et b est le plus souvent un nombre entier de 1 à 50, ou de préférence a est zéro ou un nombre entier de 1 à 25 et b est de préférence un nombre entier de 1 à 25 ; $R^3$ est le plus souvent un groupe aliphatique divalent, saturé ou insaturé, linéaire ou ramifié ayant de 2 à 20 atomes de carbone tel que par exemple un groupe alkylène, linéaire ou ramifié ayant de 2 à 20 atomes de carbone ou un groupe divalent de formule $\text{-}R^8\text{-}(\text{-X-}R^9\text{-})_c\text{-}(\text{-X-}R^{10}\text{-})_d\text{-}(\text{-X-}R^{11}\text{ -})_e\text{-}$ dans laquelle X est choisi parmi les groupes -O- et -NH-, $R^8$, $R^9$, $R^{10}$ et $R^{11}$, identiques ou différents, représentent chacun un groupe aliphatique divalent, saturé ou insaturé, linéaire ou ramifié ayant de 2 à 4 atomes de carbone, c est un nombre entier de 1 à 60, d et e, identiques ou différents, sont chacun zéro ou un nombre entier de 1 à 60 et la somme c + d + e est un nombre entier de 1 à 60 ; $R^4$ est le plus souvent un groupe aliphatique monovalent, saturé ou insaturé, linéaire ou ramifié et de préférence un groupe alcényle, linéaire ou ramifié, ou un groupe formant avec le carbone méthylènique du cycle imide, un cycle saturé ou insaturé et de préférence insaturé, ponté ou non, ayant de 5 à 10 atomes de carbone et de préférence de 6 à 8 atomes de carbone, ledit groupe $R^4$ ayant de 6 à 150, de préférence de 6 à 100 et le plus souvent de 12 à 60 atomes de carbone; n est le plus souvent un nombre de 0 à 10 et de préférence de 0 à 5.

Les composés polyazotés de la présente invention sont en particulier utilisables comme additifs multifonctionnels pour carburant moteur. Parmi ces composés, dans le cadre de leur emploi comme additifs pour carburant moteur, on utilise habituellement ceux dans lesquels le groupe $R^4$ comprend au moins 6 et de préférence au moins 12 atomes de carbone. L'addition d'une proportion mineure de ces composés à un carburant moteur permet en particulier de limiter l'encrassement de différentes parties du moteur et donc d'améliorer l'agrément de conduite en limitant et/ou en retardant l'apparition de ralenti instable et de ratés à l'allumage dans le cas

de moteur à allumage commandé.

La présente invention concerne également les compositions comprenant en poids une proportion majeure d'un carburant moteur et une proportion mineure, suffisante pour modifer au moins l'une de ses propriétés, d'au moins un composé selon la présente invention tel que décrit ci-avant ou tel que préparé selon l'une des méthodes décrites ci-après. Ces compositions comprennent le plus souvent de 10 à 10 000, et de préférence de 50 à 5000 ppm en poids d'au moins un composé selon la présente invention tel que décrit ci-avant ou tel que préparé selon l'une des méthodes décrites ci-après. Dans le cas des moteurs à allumage commandé l'utilisation des composés selon la présente invention permet en particulier de limiter la valeur de l'augmentation de l'exigence en octane du moteur au cours de son fonctionnement. Cette augmentation est le plus souvent dénommée par les hommes du métier phénomène ORI d'après l'abréviation anglo-saxonne de "Octane Requirement Increase". Dans le cas des moteurs Diesel l'utilisation des composés selon la présente invention permet en particulier de limiter l'émission de particules solides et l'émission de fumées. A titre d'exemples de carburants pouvant contenir au moins une composition selon la présente invention, on peut citer les essences telles que, par exemple, celles qui sont définies par la norme ASTM D-439, les gazoles ou carburants Diesel tels que, par exemple, ceux qui sont définis par la norme ASTM D-975. Ces carburants peuvent également contenir d'autres additifs, tels que par exemple, notamment dans le cas des carburants employés pour les moteurs à allumage commandé, des additifs antidétonants tels que des composés du plomb ( par exemple le plomb tétraéthyle), des éthers tels que le méthyltertiobutyléther ou le méthyltertioamyléther ou un mélange de méthanol et d'alcool tertiobutylique, des additifs antigivre, des additifs anticorrosion et des additifs plus spécifiquement du type détergent. Le plus souvent les compositions de la présente invention sont employées en association avec une huile minérale ou synthétique.

Les composés polyazotés de la présente invention peuvent être fabriqués par toutes méthodes connues de l'homme du métier. A titre d'exemples, non limitatifs, de méthodes permettant de préparer les composés de formule générale (I) ci-avant, on citera et on illustrera dans les exemples qui suivent les deux méthodes suivantes.

Selon la première méthode de préparation les composés de formule générale (I) peuvent être obtenus par un procédé comprenant les étapes suivantes :

a) on met en réaction, dans un solvant organique inerte, à température d'environ 60 °C à environ 160°C au moins un composé de formule générale (III)

(III) 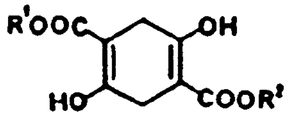

dans laquelle $R^1$ et $R^2$ ont les significations données ci-avant, avec au moins une diamine alpha-oméga biprimaire de formule générale $NH_2$-$R^3$-$NH_2$, dans un rapport molaire diamine : composé de formule générale (III) d'environ 1,1 : 1 à environ 10 : 1, de préférence d'environ 1,2 : 1 à environ 5 : 1 et par exemple 2 : 1, de manière à former le composé de formule générale (II)

(II) 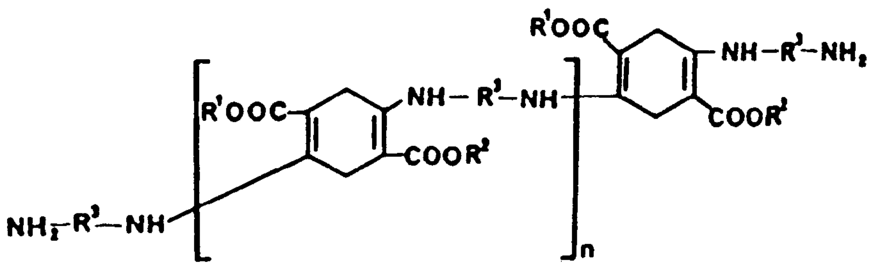

dans laquelle $R^1$, $R^2$ et $R^3$ ont les significations données ci-avant, et

b) on met en réaction le composé de formule générale (II) obtenu à l'étape a) avec un acide ou un dérivé d'un acide dicarboxylique vicinal, à une température d'environ 30 °C à environ 160°C dans des conditions de formation des cycles imides et d'élimination des produits volatils formés, la quantité d'acide ou de dérivé d'acide employée étant d'environ au moins 2 moles par mole de composé de formule générale (II) mis en réaction et on isole éventuellement, de manière classique, le composé de formule générale (I) formé.

Les esters d'acides succinosucciniques que l'on utilise le plus souvent sont des composés commerciaux

ou peuvent être obtenus facilement par les méthodes classiques de synthèse bien connu de l'homme du métier. Ces esters peuvent par exemple être obtenus par transestérification à partir du diméthylsuccinosuccinate (DMSS). A titre d'exemple non limitatifs de ces esters, on citera les esters dérivant du méthanol, de l'éthanol, des propanols, des butanols, des alcools à longue chaîne, primaires ou secondaires, tels que l'éthyl-2 hexanol, des monoéthers alkyliques de glycol ou de polyalkylèneglycols tels que les monoéthers d'alkyle de polypropylèneglycol, les monoéthers d'alkyle de polyéthylèneglycol et les monoéthers d'alkyle de polypropylène et éthylèneglycol. Le groupe alkyle de ces produits contient le plus souvent au moins 5 atomes de carbone et il est le plus souvent linéaire. A titre d'exemples de groupe alkyle, on peut citer les groupes n-pentyle et n-heptyle. Ces produits oxyalkylés sont des produits commerciaux vendus par la société SHELL sous le nom générique OXYLUBE ou par la société ICI. Ces composés ont habituellement une masse moléculaire d'environ 500 à environ 2500 et le plus souvent d'environ 600 à environ 2000. A titre d'exemple de ces composés on peut citer ceux qui sont vendus par la société ICI ayant une structure bloc du type $R^5$-O- + q (oxyde de propylène) + p (oxyde d'éthylène) dans laquelle $R^5$ représente un groupe alkyle ayant de 1 à 20 atomes de carbone, q est le nombre d'unité d'oxyde de propylène et p est le nombre d'unité d'oxyde d'éthylène.

Les diamines alpha-oméga biprimaires que l'on emploie habituellement sont des composés bien connus de l'homme du métier. Comme composés spécifiques, on peut citer à titre d'exemples non limitatifs, : l'éthylènediamine, la propylènediamine, la diéthylènetriamine, la dipropylènetriamine, la triéthylènetétramine, la tripropylènetétramine, la tétraéthylènepentamine, la tétrapropylènepentamine, l'hexaméthylènediamine, la di(triméthylène)-triamine, la diméthyl-2,2 propanediamine-1,3, la N,N'-bis(amino-3 propyl)-éthylènediamine, la (amino-2 éthyl)-amino-3 propylamine, les triméthyl-hexaméthylènediamines, pour le cas des amines ne contenant pas d'atomes d'oxygène dans leur formule. Dans le cas d'amines contenant des atomes d'oxygène dans leur formule, on peut citer les polyamines de formule

$$NH_2\text{-}R^8\text{-}(\text{-}O\text{-}R^9\text{-})_c\text{-}(\text{-}O\text{-}R^{10}\text{-})_d\text{-}(\text{-}OR^{11}\text{-})_e\text{-}NH_2$$

dans laquelle de préférence $R^8$, $R^9$ $R^{10}$ et $R^{11}$, identiques ou différents, représentent chacun un groupe alkylidène ayant de 2 à 4 atomes de carbone, par exemple éthylidène, propylidène, isopropylidène, butylidène ou isobutylidène; de préférence c est un nombre entier de 1 à 60 et d et e sont égaux à zéro; ou c est un nombre entier de 1 à 59, e est zéro ou un nombre entier tel que la somme c +e soit de 1 à 59 et d est un nombre entier de 1 à 50; avec dans chaque cas la somme c+ d + e égale à un nombre entier de 1 à 60.

Comme diamines spécifiques, on peut citer celles répondant aux formules:

(A1) $$NH_2\text{-}CH_2\text{-}CH_2\text{-}(O\text{-}CH_2\text{-}CH_2\text{-})_c\text{-}NH_2$$

(A2) 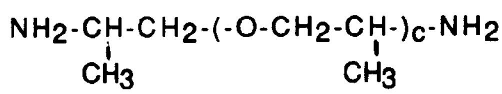

dans lesquelles c est 2, 3, 5, 6 ou environ 33
ou à la formule

(A3) 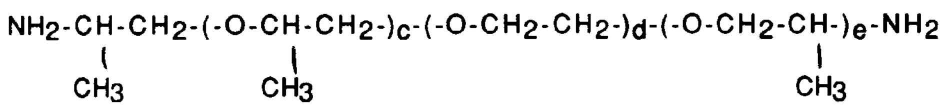

dans laquelle d est environ égal à 8, 9, 15, 16 ou 40 et c + e est environ 2 ou 3.

Ces produits sont en particulier commercialisés par la société TEXACO Chemical sous le nom Jeffamine EDR 148 pour le produit de formule (A1) dans laquelle c = 2, Jeffamine D-230 pour un produit de formule (A2) de masse moléculaire moyenne en nombre de 230, Jeffamine D-400 pour un produit de formule (A2) de masse moléculaire moyenne en nombre de 400, Jeffamine D-2000 pour un produit de formule (A2) de masse moléculaire moyenne en nombre de 2000, Jeffamine ED-600 pour un produit de formule (A3) de masse moléculaire moyenne en nombre de 600, Jeffamine ED-900 pour un produit de formule (A3) de masse moléculaire moyenne en nombre de 900 et Jeffamine ED-2001 pour un produit de formule (A3) de masse moléculaire moyenne en nombre de 2000.

L'acide ou le dérivé d'acide utilisé habituellement dans le cadre de la présente invention est un composé succinique ou un composé alkylsuccinique ou alcénylsuccinique de préférence un anhydride de type succinique. On peut également employer un dérivé phtalique, de préférence l'anhydride phtalique ou un anhydride phtalique comportant un groupe alkyle sur l'un des atomes de carbone du noyau. A titre d'exemples de composés de type succinique, on peut citer l'anhydride succinique, l'anhydride méthylsuccinique, souvent dénommé anhydride citraconique, et les anhydrides alkylsucciniques ou alcénylsucciniques ayant habituellement une

masse moléculaire moyenne en nombre d'environ 200 à 3 000, de préférence 500 à 2 000 et le plus souvent 700 à 1 500. Ces dérivés succiniques sont largement décrits dans l'art antérieur ; ils sont par exemple obtenus par l'action d'au moins une oléfine alpha ou d'un hydrocarbure chloré sur l'acide ou l'anhydride maléique. L'oléfine alpha ou l'hydrocarbure chloré utilisé dans cette synthèse peuvent être linéaires ou ramifés, et comportent habituellement de 10 à 150 atomes de carbone, de préférence de 15 à 80 atomes de carbone et le plus souvent de 20 à 75 atomes de carbone dans leur molécule. Cette oléfine peut également être un oligomère, par exemple un dimère, un trimère ou un tétramère, ou un polymère d'une oléfine inférieure, ayant par exemple de 2 à 10 atomes de carbone, telle que l'éthylène, le propylène, le n-butène-1, l'isobutène, le n-hexène-1, le n-octène-1, le méthyl-2-heptène-1 ou le méthyl-2-propyl-5-hexène-1. Il est possible d'utiliser des mélanges d'oléfines ou des mélanges d'hydrocarbures chlorés.

A titre d'exemples d'anhydrides succiniques, on peut citer l'anhydride n-octadécénylsuccinique, l'anhydride dodécénylsuccinique et les anhydrides polyisobuténylsucciniques, souvent dénommés PIBSA, ayant une masse moléculaire moyenne en nombre telle que définie ci-avant.

Selon la deuxième méthode de préparation, les composés de formule générale (I), en particulier ceux dans lesquels n est égal à zéro, peuvent être obtenus par un procédé comprenant les étapes suivantes :

a) on met en réaction, au moins une diamine alpha-oméga biprimaire de formule générale $NH_2$-$R^3$-$NH_2$, avec un acide ou un dérivé d'un acide dicarboxylique vicinal, à une température d'environ 30 °C à environ 160°C dans des conditions de formation des cycles imides et d'élimination des produits volatils formés, la quantité d'acide ou de dérivé d'acide employée étant d'environ 1 mole par mole de diamine, de manière à former le composé de formule générale (IV)

(IV) 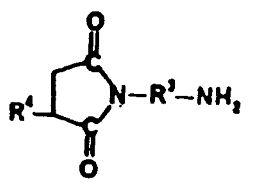

dans laquelle $R^3$ et $R^4$ ont les significations données ci-avant et

b) on met en réaction le composé de formule générale (IV) obtenu à l'étape a) avec le composé de formule générale (III), dans un rapport molaire d'environ 2 moles de composé de formule générale (IV) par mole de composé de formule générale (III), dans des conditions de formation d'un composé de formule générale (I) dans laquelle n = 0.

Dans le cadre de la présente invention on peut utiliser, pour la synthèse des produits de formule (I), (II) ou (IV),une ou plusieurs diamines biprimaires.

Les exemples suivants illustrent l'invention sans en limiter la portée.

## Exemple 1

### a) Première étape

Dans un réacteur de 2 litres à double enveloppe, muni d'un système d'agitation, d'un tube plongeant permettant une introduction d'argon, d'un thermomètre et d'un réfrigérant, on introduit sous agitation 784,3 grammes(g) (3,44 moles) de diméthylsuccinosuccinate (DMSS) et 984,1 g (7,57 moles) d'éthyl-2 hexanol. La température est portée à 135 °C puis on introduit 11,6 g (3,4x$10^{-2}$ mole) de titanate de butyle Ti-$(OC_4H_9)_4$, et on élève ensuite la température à 145 °C en maintenant l'agitation. On maintient sous agitation la température à 145 °C pendant une heure et trente minutes . On recueille une première fraction de méthanol à la pression atmosphérique puis on diminue progressivement la pression à l'aide d'une trompe à eau jusqu'à une valeur égale à 27 KiloPascals (KPa) et on recueille, (la température du ballon étant maintenue à 145 °C) après condensation, 220,6 g d'une phase alcoolique. Une analyse par chromatographie en phase gazeuse montre que la phase alcoolique ainsi récupérée contient du méthanol, de l'éthyl-2 hexanol et du butanol. La quantité totale de méthanol récupéré correspond à la quantité attendue. Le réacteur contient 1556 g de produits, qui, d'après une analyse par chromatographie de perméation sur gel, contiennent 89,4 % de succinosuccinate de di-éthyl-2 héxyle soit 1391 g (3,28 moles) ce qui correspond à une conversion molaire du DMSS de 95 %. Par distillation sous une pression partielle de 270 Pa et à la température de 120 °C on élimine les alcools résiduels. Le produit obtenu, est dissous dans du xylène avec un rapport pondéral de 1 : 1 .La solution ainsi obtenue est appelée solution N° 1.

b) deuxième étape

Dans un réacteur de 2 litres, à double enveloppe, muni d'un système d'agitation, d'une ampoule de coulée, d'un thermomètre et d'un séparateur de Dean-Stark, on introduit 296,8 g de solution 1 (0,35 mole du diester de l'acide succinosuccinique et de l'éthyl-2 hexanol). A température ambiante sous agitation, on ajoute goutte à goutte 560 g d'une solution dans le xylène (rapport pondéral xylène : produit de 1 : 1) d'une polyoxypropylènediamine de masse moléculaire 400 (Produit vendu par la société TEXACO sous le nom de Jeffamine D400), soit 0,7 mole d'amine. La température est élevée progressivement jusqu'à 120 °C et on recueille 12,5 millilitres (ml) d'eau soit 99,2 % de la quantité théorique pour la formation d'un produit de formule (II) (2 moles d'eau par mole d'ester). On récupère dans le réacteur 791 g dune solution dite solution N° 2 . Le produit obtenu est caractérisé par les méthodes classiques d'analyse. Le spectre infrarouge montre des bandes intenses à 1663 $cm^{-1}$, à 1603 $cm^{-1}$ et à 1240 $cm^{-1}$ attribuables aux fonctions esters et à la double liaison énamine. La structure à été confirmée par résonance magnétique nucléaire. La masse moléculaire moyenne en nombre du produit, mesurée par tonométrie, est de 2036.

c)Troisième étape

Dans un réacteur identique à celui utilisé dans la deuxième étape, on introduit 210 g de la solution N° 2, soit 0,0884 mole de produit de formule (II). A température ambiante sous agitation, on fait couler goutte à goutte 295,2 g d'une solution ( à 1 : 1 en poids) dans le xylène d'anhydride polyisobutènesuccinique à 0,12 fonction anhydride pour 100 g, soit 0,177 mole. La température est élevée progressivement au reflux du xylène (140 °C) . On recueille après 2 heures de chauffage 3,17 ml d'eau soit approximativement la quantité théorique correspondant à la formation de cycles imides. On récupère 497 g d'une solution à 50 % en poids de produit dans le xylène. Cette solution est appelée additif 1. L'additif 1 a été analysé après évaporation du solvant. Sa masse moléculaire moyenne en nombre mesurée par tonométrie, est de 3500. Le spectre infrarouge montre les bandes caractéristiques suivantes : 1610 $cm^{-1}$attribuable à la double liaison énamine, 1660 $cm^{-1}$ attribuable à la liaison carbonyle de l'ester de l'acide succinosuccinique, et le doublet caractéristique des succinimides aliphatiques à 1710 $cm^{-1}$ et 1770 $cm^{-1}$.

**Exemple 2**

a) Première étape

Dans un réacteur de 2 litres à double enveloppe, muni d'un système d'agitation, d'une ampoule de coulée, d'un thermomètre et d'un séparateur de Dean-Stark, on introduit 160 g d'une solution dans le xylène, à 50 % en poids, de polyoxypropylène-diamine de masse moléculaire 400 (Produit vendu par la société TEXACO sous le nom Jeffamine D400), soit 0,2 mole d'amine. A température ambiante, sous agitation, on fait couler goutte à goutte 333 g d'une solution, à 50 % en poids, dans le xylène, d'anhydride polyisobutènesuccinique à 0,12 fonction anhydride pour 100 g, soit 0,2 mole. La température est ensuite élevée progressivement au reflux du xylène (140 °C.) Après 2 heures de réaction à 140 °C, on a recueilli 3,6 g d'eau ce qui correspond à la quantité théorique pour la formation d'un cycle succinimide. On récupère dans le réacteur 487 g d'une solution dite solution N° 3. Le produit obtenu est caractérisé par les méthodes classiques d'analyse.

b) Deuxième étape

Dans un réacteur identique à celui utilisé dans la première étape, on introduit 84,8 g de la solution N° 1 préparée au cours de la première étape de l'exemple 1, soit 0,1 mole du diester de l'acide succinosuccinique et de l'éthyl-2 hexanol. A température ambiante sous agitation, on ajoute goutte à goutte les 487 g de la solution N° 3 obtenue au cours de la première étape. La température est élevée progressivement à 120 °C et on recueille 3,5 millilitres (ml) d'eau, soit 97 % de la quantité théorique pour la formation d'un produit de formule (I) (2 moles d'eau par mole de diester). dans laquelle n=0. On récupère 568 g d'une solution à 50 % en poids de produit dans le xylène. Cette solution est appelée additif 2. L'additif 2 a été analysé après évaporation du solvant. Sa masse moléculaire moyenne en nombre, mesurée par tonométrie, est de 2800. Le spectre infrarouge montre les bandes caractéristiques suivantes : 1610 $cm^{-1}$ attribuable à la double liaison énamine, 1660 $cm^{-1}$ attribuable à la liaison carbonyle de l'ester de l'acide succinosuccinique, et le doublet caractéristique des succinimides aliphatiques à 1710 $cm^{-1}$ et 1770 $cm^{-1}$.

Exemple 3

La procédure décrite dans l'exemple 1 est répétée en utilisant dans la deuxième étape une polyoxypropylène diamine de masse moléculaire 2000 (Produit vendu par la société TEXACO sous le nom de Jeffamine D2000). On a utilisé 106 g (0,125 mole) de la solution N° 1 et 1000 g (0,25 mole) d'une solution à 50 % en poids de Jeffamine D2000 dans le xylène. Le reflux est maintenu pendant le temps nécessaire pour recueillir la quantité d'eau théorique pour la formation d'un produit de formule (II). On obtient une solution N° 4 dont on prélève 281,2 g (0,0415 mole de produit de formule (II)) pour la troisième étape dans laquelle on utilise 138,4 g (0,083 mole d'anhydride) de la solution, à 50 % en poids, dans le xylène, d'anhydride polyisobutènesuccinique à 0,12 fonction anhydride pour 100 g. On récupère une solution à 50 % en poids de produit dans le xylène. Cette solution est appelée additif 3.

**Exemple 4**

La procédure de L'exemple 3 est répétée en utilisant dans la troisième étape un anhydride polyisobutènesuccinique à 0,074 fonction anhydride pour 100 g. On met en oeuvre 240 g de solution N° 4 et 191,6 g (0,0708 mole) de solution à 50 % en poids de cet anhydride polyisobutènesuccinique. On obtient ainsi une solution à 50 % en poids de produit dans le xylène. Cette solution est appelée additif 4. L'additif 4 a été analysé après évaporation du solvant. Sa masse moléculaire moyenne en nombre, mesurée par tonométrie, est de 7200.

**Exemple 5**

La procédure de l'exemple 3 est répétée en utilisant dans la troisième étape l'anhydride tétrapropénylsuccinique (ATPS). On met en oeuvre 313,7 g (0,0463 mole de produit) de solution N° 4 et 49,2 g de solution à 50 % en poids dans le xylène d'ATPS. On obtient ainsi une solution à 50 % en poids de produit dans le xylène. Cette solution est appelée additif 5.

**Exemple 6**

La procédure décrite dans l'exemple 1 est répétée en utilisant dans la première étape, à la place de l'éthyl-2 hexanol, un monoalcool polyoxypropylé et éthoxylé (vendu par la société ICI) à 70 % de fonction alcool primaire et dont la masse moléculaire est de 1097 . On utilise 182,4 g (0,8 mole) de DMSS et 2512 g (2,29 moles) d'alcool polyoxyalkylé (soit un excès de 30 %). On obtient, après dilution dans du xylène, une solution à 50 % en poids de produit, appelée solution N° 5. On prélève une quantité aliquote de cette solution N° 5 correspondant à 0,3 mole de DMSS de départ. On fait réagir cette quantité aliquote de solution N° 5 dans les conditions décrites pour la deuxième étape dans l'exemple 1 avec 440 g d'une solution à 50 % en poids dans le xylène de Jeffamine D400 soit 0,6 mole. La quantité d'eau recueillie correspond à la quantité théorique attendue pour la formation d'un produit de formule (II) (2 moles d'eau par mole de diester). On obtient ainsi 2195 g d'une solution appelée solution N° 6. On prélève une partie de cette solution N° 6, correspondant à 0,0425 mole de DMSS soit 0,085 fonction amine primaire, que l'on met en réaction, dans les conditions décrites en liaison avec l'étape 3 de l'exemple 1, avec 141,7 g d'une solution, à 50 % en poids dans le xylène, d'anhydride polyisobutènesuccinique à 0,12 fonction pour 100 g soit 0,085 mole. On récupère une solution à 50 % en poids de produit dans le xylène. Cette solution est appelée additif 6.

**Exemple 7**

La procédure décrite dans l'exemple 6 est répétée en utilisant dans la troisième étape un anhydride polyisobutènesuccinique à 0,074 fonction anhydride pour 100 g. On emploie la même quantité de solution N° 6 que dans l'exemple 6 (soit 0,085 fonction amine primaire) et 230 g d'une solution à 50 % en poids dans le xylène de l'anhydride soit 0,085 mole. On récupère une solution à 50 % en poids de produit dans le xylène. Cette solution est appelée additif 7.

**Exemple 8**

La procédure décrite dans l'exemple 2 est répétée en utilisant, dans la première étape, sous forme de solution à 50 % en poids dans le xylène, 0,2 mole de tétraéthylènepentamine et 0,2 mole d'anhydride polyisobutènesuccinique à 0,074 fonction pour 100 g. Dans la deuxième étape on met en réaction 0,1 mole de DMSS avec 0,29 mole de l'alcool polyoxyalkylé utilisé dans l'exemple 6 dans les conditions décrites ci-avant pour la

transestérification. La troisième étape est effectuée dans les mêmes conditions que celles qui sont décrites en liaison avec l'exemple 2. On obtient une solution dans le xylène que l'on ajuste à 50 % en poids de produit. Cette solution à 50 % en poids de produit est appelée additif 8.

**Exemple 9**

La procédure décrite dans l'exemple 8 est répétée en remplaçant la tétraéthylènepentamine par l'hexaméthylènediamine. On obtient une solution dans le xylène qui, ajustée à 50 % en poids de produit, constitue l'additif 9.

Les additifs préparés dans les exemples 3 à 9 présentent tous, en spectroscopie infrarouge, les mêmes bandes caractéristiques que les additifs obtenus dans les exemples 1 et 2.

**Exemple 10**

Une série d'essais est effectuée de manière à évaluer les propriétés de limitation de l'augmentation de l'exigence en octane d'un moteur alimenté avec un carburant seul, un carburant contenant l'un des additifs selon l'invention et renfermant également un additif détergent classique (C) et avec un carburant contenant l'additif C mais ne contenant pas d'additif anti ORI. Le carburant utilisé est un carburant sans plomb comprenant en volume:

- 26 % d'aromatiques
- 6,3 % d'oléfines et
- 67,7 % de composés saturés (paraffines et naphténiques)

Les additifs anti ORI selon l'invention ont été ajoutés au carburant de manière à avoir une concentration en poids de 100 ppm. Les essais ont été effectués sur un banc moteur équipé d'un moteur Renault de type F 2 N ayant une cylindrée de 1721 $cm^3$ et un taux de compression de 9,5. Ces essais ont été effectués en suivant la procédure Renault 22700 modifiée, avec une température de l'eau en sortie de culasse de 95 ° C à plus ou moins 2 degrés et une température de l'huile au niveau de la rampe de 140 °C. Le cycle d'essai dure 12 heures (h) et comprend :

- 1 h de ralenti à vide,
- 4 h à 2500 tours par minutes (t/min) à la moitié de la pleine charge,
- 3 h à 3500 t/min à vide,
- 4 h à 2500 tours par minutes (t/min) à la moitié de la pleine charge.

Les valeurs d'avance correspondant à l'apparition du cliquetis, et exprimées en degrés de vilebrequin et désignées très souvent par les initiales KLSA (initiales anglo-saxonnes de "Knock Limit Spark Advance") sont déterminées 1 fois à 0 et 150 heures à différents régimes du moteur. Les résultats obtenus sont exprimés en variations de KLSA à 150 heures pour quatre régimes différents du moteur : 2000 t/min, 2900 t/min, 3600 t/min et 4100 t/min. Ces résultats sont présentés dans le tableau 1 ci-après . Le poids global des dépôts sur les soupapes d'admission a également été mesuré et les résultats sont donnés dans le tableau 1. Ces résultats montrent : d'une part que les additifs selon l'invention donnent des variations plus faible de KLSA, limitent l'augmentation de l'exigence en octane du moteur et retardent l'apparition de ralenti instable ; d'autre part que le poids de dépôts sur les soupapes d'admission est largement diminué par rapport à ce que l'on obtient avec le carburant seul ou avec le carburant contenant l'additif détergent C.

## TABLEAU 1

| Δ KLSA à | carburant seul | carburant + additif C sans anti ORI | carburant + additif C + additif 4 | carburant + additif C + additif 6 |
|---|---|---|---|---|
| 2000 t/min | 5 | 8 | 3 | 4 |
| 2900 t/min | 4 | 10 | 3 | 3 |
| 3600 t/min | 4 | 16 | 2 | 3 |
| 4100 t/min | 7 | 15 | 6 | 6 |
| dépôts en g | 2,01 | 1,20 | 0,91 | 1,18 |

**Exemple 11**

Des essais sont réalisés de façon à évaluer les propriétés anti-fumées des additifs selon l'invention dans un carburant Diesel. Le carburant Diesel utilisé a les principales caractéristiques suivantes :

- Température limite de filtrabilité : -3 °C
- Point initial de distillation : 162 °C
- Point 95 %de distillation : 366 °C
- Masse volumique à 15 °C : 0,8331
- Indice de cétane calculé : 50,4

La quantité d'additif est ajoutée au carburant de manière à obtenir une concentration, en poids de matière active dans le carburant additivé, de 100 ppm.

Les essais sont effectués sur moteur RENAULT F 8 Q suivant la procédure décrite ci-après . Après démarrage, on procédé à la mise en température du moteur en régulant notamment la température d'eau de refroidissement à 85 °C. Le régime moteur est fixé alors à 2500 tours par minutes sous demi-charges pendant environ 15 minutes avant d'effectuer la mesure de l'indice de fumée. Cette mesure est réalisée suivant la méthode BOSCH bien connue de l'homme du métier.Le tableau 2 rassemble les résultats obtenus avec le carburant non additivé et avec le carburant contenant l'additif 4.

## TABLEAU 2

| Carburant | Indice de fumée BOSCH |
|---|---|
| Carburant seul | 2,3 |
| Carburant + additif 4 | 0,8 |

Ces résultats montrent que l'additif 4 préparé dans l'exemple 4 permet de réduire notablement le niveau des fumées émises à l'échappement d'un moteur Diesel.

**Revendications**

**1-** Composé polyazoté, comportant deux cycles terminaux de type imide, répondant à la formule générale (I) :

(I)

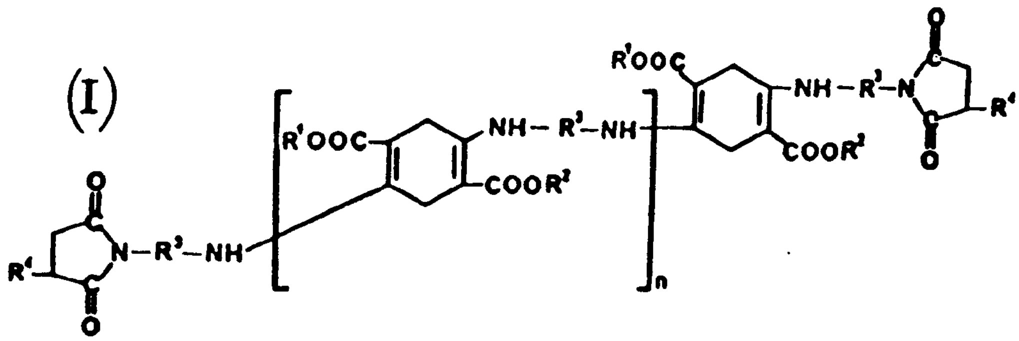

dans laquelle $R^1$ et $R^2$, identiques ou différents, représentent chacun un groupe hydrocarboné ayant de 1 à 120 atomes de carbone ou un groupe de formule $R^5\text{-}(\text{-O-}R^6\text{-})_a\text{-}(\text{-O-}R^7\text{-})_b\text{-}$ dans laquelle $R^6$ et $R^7$, identiques ou différents, représentent chacun un groupe hydrocarboné divalent ayant de 2 à 6 atomes de carbone, $R^5$ représente un groupe hydrocarboné monovalent ayant de 1 à 60 atomes de carbone ; a est zéro ou un nombre entier de 1 à 100 et b est un nombre entier de 1 à 100; $R^3$ est un groupe hydrocarboné divalent ayant de 2 à 60 atomes de carbone ou un groupe divalent de formule $\text{-}R^8\text{-}(\text{-X-}R^9\text{-})_c\text{-}(\text{-X-}R^{10}\text{-})_d\text{-}(\text{-X-}R^{11}\text{-})_e\text{-}$ dans laquelle X est choisi parmi les groupes -O- et $\text{-NR}^{12}\text{-}$, $R^{12}$ représentant un atome d'hydrogène ou un groupe hydrocarboné ayant de 1 à 6 atomes de carbone, $R^8$, $R^9$, $R^{10}$ et $R^{11}$, identiques ou différents, représentent chacun un groupe hydrocarboné divalent ayant de 2 à 6 atomes de carbone, c est un nombre entier de 1 à 120, d et e, identiques ou différents, sont chacun zéro ou un nombre entier de 1 à 120 et la somme c + d + e est un nombre entier de 1 à 120; $R^4$ est atome d'hydrogène ou un groupe hydrocarboné ayant de 1 à 200 atomes de carbone et n est un nombre de 0 à 20.

**2-** Composé de formule générale (I) selon la revendication 1 dans laquelle $R^1$ et $R^2$, identiques ou différents, représentent chacun un groupe aliphatique monovalent, saturé ou insaturé, linéaire ou ramifié ayant de : à 60 atomes de carbone ou un groupe de formule $R^5\text{-}(\text{-O-}R^6\text{-})_a\text{-}(\text{-O-}R^7\text{-})_b\text{-}$ dans laquelle $R^6$ et $R^7$, identiques ou différents, représentent chacun un groupe aliphatique divalent, saturé ou insaturé, linéaire ou ramifié ayant de 2 à 4 atomes de carbone, $R^5$ représente un groupe aliphatique monovalent, saturé ou insaturé, linéaire ou ramifié ayant de 1 à 20 atomes de carbone, a est zéro ou un nombre entier de 1 à 50 et b est un nombre entier de 1 à 50 ; $R^3$ est un groupe aliphatique divalent, saturé ou insaturé, linéaire ou ramifié ayant de 2 à 20 atomes de carbone ou un groupe divalent de formule

$$\text{-}R^8\text{-}(\text{-X-}R^9\text{-})_c\text{-}(\text{-X-}R^{10}\text{-})_d\text{-}(\text{-X-}R^{11}\text{-})_e$$

dans laquelle X est choisi parmi les groupes -O- et -NH-, $R^8$, $R^9$, $R^{10}$ et $R^{11}$, identiques ou différents, représentent chacun un groupe aliphatique divalent, saturé ou insaturé, linéaire ou ramifié ayant de 2 à 4 atomes de carbone, c est un nombre entier de 1 à 60 , d et e, identiques ou différents, sont chacun zéro ou un nombre entier de 1 à 60 et la somme c + d + e est un nombre entier de 1 à 60 ; $R^4$ est un groupe aliphatique monovalent, saturé ou insaturé, linéaire ou ramifié ou un groupe formant avec le carbone méthylènique du cycle imide, un cycle saturé ou insaturé, ponte ou non, comportant de 5 à 10 atomes de carbone, ledit groupe $R^4$ ayant de 6 à 150 atomes de carbone, et n est un nombre de 0 à 10.

**3-** Composé de formule générale (I) selon la revendication 1 ou 2 dans laquelle $R^1$ et $R^2$, identiques ou différents, représentent chacun un groupe alcoyle, linéaire ou ramifié ayant de 1 à 30 atomes de carbone ou un groupe de formule $R^5\text{-}(\text{-O-}R^6\text{-})_a\text{-}(\text{-O-}R^7\text{-})_b\text{-}$ dans laquelle $R^6$ et $R^7$, identiques ou différents, représentent chacun un groupe alkylène, linéaire ou ramifié ayant de 2 à 4 atomes de carbone, $R^5$ est un groupe alkyle, linéaire ou ramifié ayant de 1 à 20 atomes de carbone, a est zéro ou un nombre entier de 1 à 25 et b est un nombre entier de 1 à 25 ; $R^3$ est un groupe alkylène, linéaire ou ramifié ayant de 2 à 20 atomes de carbone ; $R^4$ est un groupe alcènyle, linéaire ou ramifié, ou un groupe formant avec le carbone méthylénique du cycle imide un cycle insaturé, ponté ou non, comportant de 6 à 8 atomes de carbone, ledit groupe $R^4$ ayant de 6 à 100 atomes de carbone et n est un nombre de 0 à 5.

**4-** Composé de formule générale (I) selon la revendication 1 ou 2 dans laquelle $R^1$ et $R^2$, identiques ou différents, représentent chacun un groupe alkyle, linéaire ou ramifié ayant de 1 à 30 atomes de carbone ou un groupe de formule $R^5\text{-}(\text{-O-}R^6\text{-})_a\text{-}(\text{-O-}R^7\text{-})_b\text{-}$dans laquelle $R^6$ et $R^7$, identiques ou différents, représentent chacun un groupe alkyle, linéaire ou ramifié ayant de 2 à 4 atomes de carbone, $R^5$ est un groupe alkyle, linéaire ou ramifié ayant de 1 à 20 atomes de carbone, a est zéro ou un nombre entier de 1 à 25 et b est un nombre entier de 1 à 25 ; R3 est un groupe divalent de formule

$$\text{-}R^8\text{-}(\text{-X-}R^9\text{-})_c\text{-}(\text{-X-}R^{10}\text{-})_d\text{-}(\text{-X-}R^{11}\text{-})_e\text{-}$$

dans laquelle X est choisi parmi les groupes -O- et -NH-, $R^8$, $R^9$, $R^{10}$ et $R^{11}$, identiques ou différents, repré-

sentent chacun un groupe aliphatique, saturé ou insaturé, linéaire ou ramifié ayant de 2 à 4 atomes de carbone, c est un nombre entier de 1 à 60, d et e, identiques ou différents, sont chacun zéro ou un nombre entier de 1 à 60 et la somme c + d + e est un nombre entier de 1 à 60 ; $R^4$ est un groupe alcényle, linéaire ou ramifié, ou un groupe formant avec le carbone méthylénique du cycle imide un cycle, ponté ou non, insaturé, comportant de 6 à 8 atomes de carbone, ledit groupe $R^4$ ayant de 6 à 100 atomes de carbone et n est un nombre de 0 à 5.

**5-** Composé de formule générale (I) selon la revendication 1 ou 2 dans laquelle $R^1$ et $R^2$, identiques ou différents, représentent chacun un groupe aliphatique, saturé ou insaturé, linéaire ou ramifié ayant de 1 à 60 atomes de carbone, $R^3$ est un groupe divalent de formule $-R^8-(-X-R^9-)_c-(-X-R^{10}-)_d-(-X-R^{11}-)_e-$ dans laquelle X est choisi parmi les groupes -O- et -NH-, $R^8$, $R^9$, $R^{10}$, et $R^{11}$, identiques ou différents, représentent chacun un groupe aliphatique, saturé ou insaturé, linéaire ou ramifié ayant de 2 à 4 atomes de carbone, c est un nombre entier de 1 à 60, d et e, identiques ou différents, sont chacun zéro ou un nombre entier de 1 à 60 et la somme c + d + e est un nombre entier de 1 à 60 ; $R^4$ est un groupe alcényle, linéaire ou ramifié, ou un groupe formant avec le carbone méthylènique du cycle imide un cycle, ponté ou non, insaturé, comportant de 6 à 8 atomes de carbone, ledit groupe $R^4$ ayant de 6 à 100 atomes de carbone et n est un nombre de 0 à 5.

**6-** Composé de formule générale (I) selon la revendication 1 ou 2 dans laquelle $R^1$ et $R^2$, identiques ou différents, représentent chacun un groupe de formule

$$R^5-(-O-R^6-)_a-(-OR^7-)_b-$$

dans laquelle $R^6$ et $R^7$, identiques ou différents, représentent chacun un groupe aliphatique divalent, saturé ou insaturé, linéaire ou ramifié divalent ayant de 2 à 4 atomes de carbone, $R^5$ représente un groupe aliphatique, saturé ou insaturé, linéaire ou ramifié ayant de 1 à 20 atomes de carbone ; $R^3$ est un groupe divalent de formule $-R^8-(-X-R^9-)_c-(-X-R^{10}-)_d-(-X-R^{11}-)_e-$ dans laquelle X est choisi parmi les groupes -O- et -NH-, $R^8$, $R^9$, $R^{10}$ et $R^{11}$, identiques ou différents, représentent chacun un groupe aliphatique, saturé ou insaturé, linéaire ou ramifié ayant de 2 à 4 atomes de carbone, c est un nombre entier de 1 à 60, d et e, identiques ou différents, sont chacun zéro ou un nombre entier de 1 à 60 et la somme c + d + e est un nombre entier de 1 à 60 ; $R^4$ est un groupe alcènyle, linéaire ou ramifié, ou un groupe formant avec le carbone méthylènique du cycle imide un cycle insaturé, ponté ou non, comportant de 6 à 8 atomes de carbone, ledit groupe $R^4$ ayant de 6 à 100 atomes de carbone et n est un nombre de 0 à 5.

**7-** Composé de formule générale (I) selon l'une des revendications 4 à 6 dans laquelle $R^3$ est un groupe divalent de formule $-R^8-(-X-R^9-)_c-(-X-R^{10}-)_d-(-X-R^{11}-)_e-$ dans laquelle X est le groupe -O-, $R^8$, $R^9$, $R^{10}$ et $R^{11}$, identiques ou différents, représentent chacun un groupe aliphatique, saturé ou insaturé, linéaire ou ramifié ayant de 2 à 4 atomes de carbone, c est un nombre entier de 1 à 60, d et e, identiques ou différents, sont chacun zéro ou un nombre entier de 1 à 60 et la somme c + d + e est un nombre entier de 1 à 60.

**8-** Composé de formule générale (I) selon l'une des revendications 4 à 6 dans laquelle $R^3$ est un groupe divalent de formule $-R^8-(-X-R^9-)_c-(-X-R^{10}-)_d-(-X-R^{11}-)_e-$ dans laquelle X est le groupe -NH-, $R^8$, $R^9$, $R^{10}$ et $R^{11}$, identiques ou différents, représentent chacun un groupe aliphatique, saturé ou insaturé, linéaire ou ramifié ayant de 2 à 4 atomes de carbone, c,d et e, identiques ou différents, sont chacun zéro ou un nombre entier de 1 à 60.

**9-** Procédé de préparation d'un composé selon l'une des revendications 1 à 8, caractérisé en ce qu'il comprend les étapes suivantes :

a)on met en réaction, dans un solvant organique inerte, à une température d'environ 60 °C à environ 160°C au moins un composé de formule générale (III)

(III) 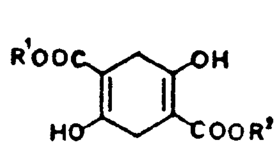

avec au moins une diamine alpha-oméga biprimaire de formule générale $NH_2-R^3-NH_2$ dans un rapport molaire diamine : composé de formule générale (III) d'environ 1,1 : 1 à environ 10 : 1, de manière à former le composé de formule générale (II)

(II)

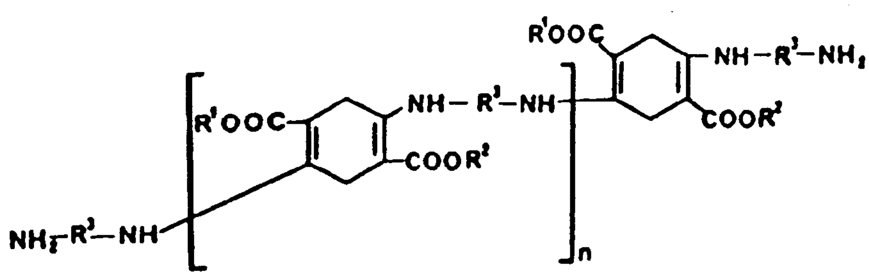

$R^1$, $R^2$ et $R^3$ ayant les mêmes significations que dans chacune des revendications 1 à 8, et

b) on met en réaction le composé de formule générale (II) obtenu à l'étape a) avec un acide ou un dérivé d'un acide dicarboxylique vicinal, à une température d'environ 30 °C à environ 160°C dans des conditions de formation des cycles imides et d'élimination des produits volatils formés, la quantité d'acide ou de dérivé d'acide employée étant d'environ au moins 2 moles par mole de composé de formule générale (II) mis en réaction.

**10-** Procédé de préparation d'un composé selon l'une des revendications 1 à 8, caractérisé en ce qu'il comprend les étapes suivantes :

a)on met en réaction, au moins une diamine alpha-oméga biprimaire de formule générale $NH_2$-$R^3$-$NH_2$, avec un acide ou un dérivé d'un acide dicarboxylique vicinal, à une température d'environ 30 °C à environ 160°C dans des conditions de formation des cycles imides et d'élimination des produits volatils formés, la quantité d'acide ou de dérivé d'acide employée étant d'environ 1 mole par mole de diamine, de manière à former le composé de formule générale (IV)

(IV)

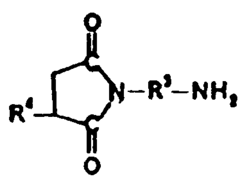

$R^3$ et $R^4$ ayant les mêmes significations que dans chacune des revendications 1 à 8, et

b) on met en réaction le composé de formule générale (IV) obtenu à l'étape a) avec le composé de formule générale (III), dans un rapport molaire d'environ 2 moles de composé de formule générale (IV) par mole de composé de formule générale (III), dans des conditions de formation d'un composé de formule générale (I) dans laquelle n = 0.

**11-** Utilisation d'un composé selon l'une des revendications 1 à 8 ou d'un composé préparé selon la revendication 9 ou 10 comme additif multifonctionnel pour carburants moteurs.

**12-** Composition comprenant en poids une proportion majeure d'un carburant moteur et une proportion mineure, suffisante pour modifier au moins l'une de ses propriétés, d'au moins un composé selon l'une des revendications 1 à 8 ou d'un composé préparé selon la revendication 9 ou 10.

**13-** Composition selon la revendication 12 comprenant de 10 à 10000, et de préférence de 50 à 5000 ppm en poids d'au moins un composé selon l'une des revendications 1 à 8 ou d'un composé préparé selon la revendication 9 ou 10.

Office européen des brevets

# RAPPORT DE RECHERCHE EUROPEENNE

Numero de la demande

EP 92 40 2134

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | DE-A-2 714 403 (SOCIETE OROGIL)<br>* revendications; exemples 7-9 *<br>--- | 1,11-13 | C07D207/40<br>C08G73/02 |
| A | DE-A-2 152 271 (TEKKOSHA CO. LTD.)<br>* page 2, formule VII *<br>--- | 1 | |
| A | CHEMICAL ABSTRACTS, vol. 83, no. 2, 14 Juillet 1975, Columbus, Ohio, US;<br>abstract no. 10953R,<br>J.A. MOORE ET AL.: 'Poly(amine esters) derived from diethyl ...'<br>page 4 ;<br>& Macromolecules 1975, 8(2), 121-7<br>--- | 1 | |
| A | CHEMICAL ABSTRACTS, vol. 76, no. 12, 20 Mars 1972, Columbus, Ohio, US;<br>abstract no. 60269Y,<br>A. TAI ET AL.: 'Structural changes in polymers of diethyl ...'<br>page 15 ;<br>& J. Polym. Sci., Part A-1 1971, 9(9), 2481-91<br>--- | 1 | **DOMAINES TECHNIQUES RECHERCHES (Int. Cl.5 )** |
| A | CHEMICAL ABSTRACTS, vol. 93, no. 23, 8 Décembre 1980, Columbus, Ohio, US;<br>abstract no. 220441E,<br>J. SINNREICH: 'Chemistry of succinylsuccinic acid derivatives ...'<br>page 500 ;<br>& Synthesis 1980, (7), 578-80<br>--- | 9 | C07D<br>C08G |
| A | US-A-3 723 460 (W.T. BRANNEN ET AL.)<br>----- | 11 | |

Le présent rapport a été établi pour toutes les revendications

| Lieu de la recherche | Date d'achèvement de la recherche | Examinateur |
|---|---|---|
| BERLIN | 06 OCTOBRE 1992 | CHRISTIAN HASS |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)